# EUROPEAN PATENT SPECIFICATION

(11) **EP 1 334 765 B1**
(45) Date of publication and mention of the grant of the patent: **30.06.2010**
(21) Application number: 01974773.2
(22) Date of filing: 11.10.2001
(51) Int. Cl.: A61K 9/127, B01J 3/00, B01J 13/02, A61J 3/07

(54) **PROCESS FOR PRODUCING LIPOSOME**
VERFAHREN ZUR HERSTELLUNG VON LIPOSOMEN
PROCEDE DE PRODUCTION DE LIPOSOMES

(30) Priority: 13.10.2000 JP 2000313599
(43) Date of publication of application: 13.08.2003
(73) Proprietor: National Institute of Advanced Industrial Science and Technology, Tokyo 100-8921 (JP); Shu Uemura Cosmetics Inc., Tokyo 107-0062 (JP); Otake, Katsuto, Tsukuba-shi, Ibaraki 305-8565 (JP); Abe, Masahiko, Noda-city, Chiba 278-0017 (JP)
(72) Inventor: OTAKE, K., 1-1-1, Higashi Tsukuba-shi Ibaraki 305 (JP); ABE, Masahiko, Noda-City, Chiba 278-0017 (JP); SAKAI, Hideki, Kamakura-shi, Kanagawa 247-0071 (JP); IMURA, Tomohiro, Tougane-shi, Chiba 283-0003 (JP); KAISE, Chihiro c/o SHU UEMURA COSMETICS INC. LAB., Setagaya-ku, Tokyo 156-0054 (JP); SAKURAI, M. c/o SHU UEMURA COSMETICS INC. LAB., Setagaya-ku, Tokyo 156-0054 (JP); ARAI, Yoichiro c/o SHU UEMURA COSMETICS INC. LAB., Setagaya-ku, Tokyo 156-0054 (JP); KANEKO, T. c/o SHU UEMURA COSMETICS INC. LAB., Setagaya-ku, Tokyo 156-0054 (JP)
(74) Representative: Ratcliffe, Susan Margaret
(86) International application number: PCT/JP2001/008907
(87) International publication number: WO 2002/032564

(56) References cited:
- WO-A1-97/14407
- US-A- 5 554 382
- US-A- 5 700 482
- US-A1- 5 554 382
- US-A1- 5 700 482

## Description

### TECHNICAL FIELD

The present invention relates to a method for producing liposomes that take advantage of supercritical carbon dioxide.

### TECHNICAL BACKGROUND

Liposomes, which are formed of a lipid bilayer, can encapsulate a variety of water-soluble substances and are therefore used as an effective carrier for delivering pharmacologically active substances into a living system.

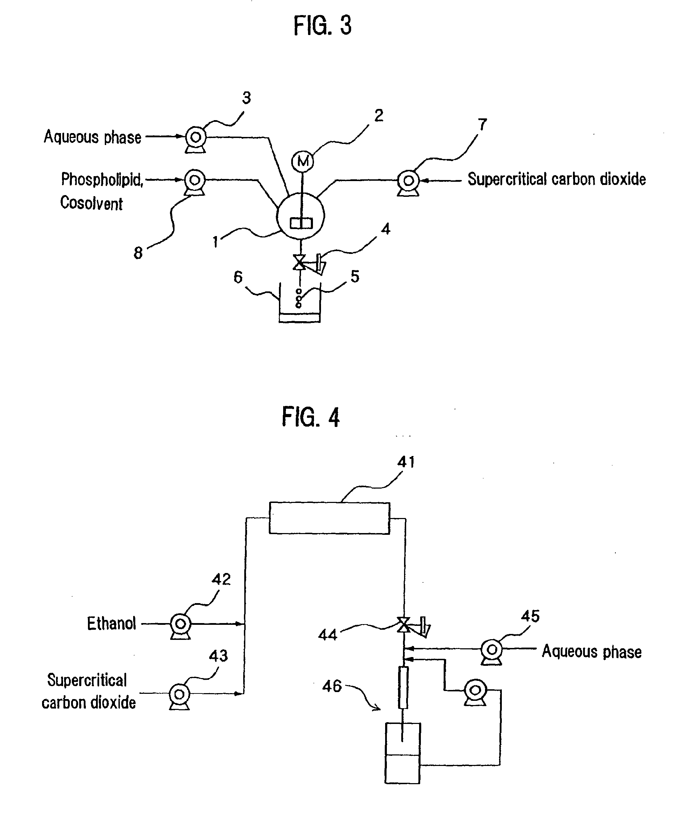

Liposomes can be obtained by subjecting suspensions of phospholipids, which are amphipathic substances, to stirring and other proper treatments. Among known techniques for preparing liposome on industrial scale are (1) sonication technique, in which a suspension of a phospholipid or glycolipid is exposed to ultrasound; (2) surfactant removal technique, which involves mixing a phospholipid or a glycolipid with a surfactant to form micelles and subsequently removing the surfactant; (3) organic solvent injection technique, in which a phospholipid or a glycolipid dissolved in an organic solvent is injected into a water reservoir so that liposomes are formed at the interface between the aqueous phase and the organic phase; (4) freeze-melt technique, which involves freezing a suspension of a phospholipid or a glycolipid in water, subsequently melting the frozen suspension to form a lipid bilayer, and further freezing and thawing the lipid bilayer to form liposomes; (5) reversed phase evaporation technique, in which a mixture of a hydrophobic organic solvent and a small amount of an aqueous solvent is sonicated to form a W/O emulsion (*i*.*e*., reversed micelles), from which the organic solvent is then removed *in vacuo*; and (6) supercritical carbon dioxide technique, in which an aqueous solution to be entrapped within liposomes is injected, while being stirred, into a phospholipid or a glycolipid dissolved in a fluid mixture of supercritical carbon dioxide and ethanol during the pressure reduction process (Japanese Patent Laid-Open Publication No. Hei 6-315624). The principle of the supercritical carbon dioxide technique, which is generally depicted in Fig. 3, is as follows: supercritical carbon dioxide and ethanol are sent by pumps 33 and 32, respectively, and are fed into a column 31 filled with a phospholipid. The phospholipid, dissolved in a fluid mixture of carbon dioxide and ethanol, is transported to a reduction valve 34, where the pressure of the fluid mixture is reduced. Once phospholipid precipitates during the pressure reduction, an aqueous phase containing a water-soluble material to be entrapped in the liposome is added to the phospholipid via a pump 35. The resulting mixture is then stirred by a mixer 36 to form liposomes within the mixer 36.

The foregoing conventional techniques are, however, each associated with its own problems such as those described below, and no practical technique or apparatus has ever been known that enables industrial-scale production of liposome:

### (1) Sonication technique

Despite its advantage that large unilamellar vesicles (SLVs) with relatively uniform size of 0.002 to 0.2µm can be prepared, the technique only permits production of liposomes with small capacity to contain water-soluble substances.

### (2) Surfactant removal technique

The drawback with this technique is that the removal of the surfactant requires dialysis.

### (3) Organic solvent injection technique

This technique only provides liposomes with a low efficiency to entrap water-soluble substances and often requires the removal of organic solvents.

### (4) Freeze-thaw technique

Though the technique provides high trapping efficiency, the freezing process used in the technique adds to the required energy cost.

### (5) Reversed-phased evaporation technique

Despite the high trapping efficiency provided by this technique, the removal of organic solvents is necessary.

### (6) Supercritical carbon dioxide technique

In the technique described in Japanese Patent Laid-Open Publication No. Hei 6-315624 or other publications, liposomes are not formed under supercritical conditions but are formed by making use of the rapid expansion of supercritical solutions (RESS). In this process, phospholipid is allowed to precipitate, while forming fine particles, as its solubility abruptly decreases upon pressure reduction, and the aqueous solution is then mixed with the phospholipid precipitates to form liposomes. Processes using supercritical carbon dioxide are furthermore described in US 5,554,382 and US 5,700,482.

As set forth, some of the conventional techniques described in (1) to (6) above require multiple steps for operation while others involve the use of organic solvents harmful to human body. In addition, these techniques require significant amounts of energy to remove organic solvents during its production.

Accordingly, it is an objective of the present invention to provide a method and an apparatus that make it possible to produce liposomes with high trapping efficiency in fewer steps and without using harmful organic solvents.

### DISCLOSURE OF THE INVENTION

During the course of their extensive studies, the present inventors have conceived of a novel approach by which liposome can be made in a single-step process by making use of supercritical carbon dioxide. The finding ultimately led the present inventors to complete the present invention.

Accordingly, the present invention provides a method for producing liposome encapsulating a desired substance, characterized in that:
an aqueous phase containing the substance to be encapsulated is added to a homogenous mixture of a phospholipid or a glycolipid and carbon dioxide either under a supercritical condition or under a condition in which temperature or pressure is higher than or equal to its critical point;
while adding the aqueous phase said condition is kept by maintaining the pressure and the homogenous fluid mixture is vigorously stirred;
and, once a predetermined amount of the aqueous phase has been added, the pressure is released to produce the liposomes.

The method of the present invention is characteristic in that it enables single-step production of liposomes encapsulating a desired substance through the addition of the aqueous phase containing the water-soluble or hydrophilic substance to be encapsulated to a homogenous mixture of a phospholipid and carbon dioxide either under a supercritical condition or under a condition in which temperature or pressure is higher than or equal to its critical point. The liposome produced by the method of the present invention has improved trapping efficiency and can thus encapsulate larger amounts of the desired substance than can the conventional liposome.

As used herein, the expression "carbon dioxide under a supercritical condition" refers to carbon dioxide under a supercritical condition, which is defined as a condition in which the temperature is higher than, or equal to, the critical temperature (*i.e*., 30.98°C) and, at the same time, the pressure is higher than, or equal to, the critical pressure (*i.e*., 7.3773±0.0030MPa). As used herein, the expression "carbon dioxide under a condition in which temperature or pressure is higher than or equal to its critical point" refers to carbon dioxide under a condition in which either the temperature is higher than, or equal to, the critical temperature or the pressure is higher than, or equal to, the critical pressure (the other parameter remains less than its critical value). Hereinafter, the carbon dioxide under a supercritical condition and the carbon dioxide under a condition in which temperature or pressure is higher than or equal to its critical point are collectively referred to as "supercritical carbon dioxide."

Phospholipid or glycolipid for use in the present invention may be any type of phospholipid or glycolipid that can form a lipid bilayer.

Phospholipid, also known as phosphatide, is a collective term for a group of compounds consisting of compound lipids including a phosphate ester and a C-P bond. Examples of phospholipid for use in the present invention include, but are not limited to, glycerophospholipids such as phosphatidylcholine, phosphatidylethanolamine, phosphatidylseline, phosphatidic acid, phosphatidylglycerol, phosphatidylinositol, cardiolipin, yolk lecithin, hydrogenated yolk lecithin, soy lecithin, and hydrogenated soy lecithin; and sphingophospholipids such as sphingomyelin, ceramide phosphorylethanolamine, and ceramide phosphorylglycerol.

Examples of glycolipid include, but are not limited to, glycerolipids such as digalactosyldiglyceride and galactosyldiglyceride sulfate; and sphingoglycolipids such as galactosylceramide, galactosylceramide sulfate, lactosylceramide, ganglioside G7, ganglioside G6, and ganglioside G4.

A cosolvent is preferably used to prepare a uniform mixture of phospholipid and supercritical carbon dioxide. The addition of the cosolvent to the system increases the solubility of the substance to be encapsulated in the supercritical carbon dioxide, which otherwise can hardly dissolve the substance. The amount of the cosolvent to be added is preferably 15wt% or less with respect to the amount of the supercritical carbon dioxide. The amount exceeding 15wt% causes the ethanol liquid phase to crystallize and thus is unfavorable. Preferably, the amount of ethanol is kept as small as possible since ethanol interferes with the formation of liposomes.

Ethanol, for example, may suitably be used as the cosolvent for that purpose.

The aqueous phase to be added to the uniform mixture of phospholipid and the supercritical carbon dioxide contains the substance to be encapsulated in liposomes.

The substance to be encapsulated for use in the present invention may be any water-soluble or hydrophilic substance.

The aqueous phase may be added in any amount that ensures the fluidity of water and does not interfere with the formation of liposomes.

The water-soluble or hydrophilic substance to be encapsulated may be a pharmacologically active ingredient.

Examples of the pharmacologically active ingredient include, but are not limited to, magnesium L-ascorbyl phosphate, glycoside ascorbate, dipotassium glycyrrhizinate, β-glycyrrhetic acid, ammonium glycyrrhetate, stearyl glycyrrhetate, esculin, pantothenyl alcohol, pantothenic acid and salts thereof, thiamine, flavin, folic acid, antibiotics, or at least one non-steroid anti-inflammatory agent, ketoprofen, ibuprofen, bufexamac, or indomethacin. The method of the present invention has made it possible for the liposomes to encapsulate larger amounts of the pharmacological active ingredients than are possible by the conventional techniques. As a result, the liposomes of the present invention improve percutaneous absorption of the active ingredient when used in an external preparation and improve absorption by body when used for internal use.

The substance to be encapsulated for use in the present invention may be a water-soluble color. Examples of the water-soluble color include, but are not limited to, red #104, red #2, red #3, blue #1, blue #2, yellow #4, yellow #5, green #3, carmine, carthamine, monascus color, gardenia color, anthocyanin, and chlorophyll. While these water-soluble colors are highly unstable in nature and are susceptible to oxidation and decoloration, they can be made stable by encapsulating in the liposomes. Ascorbic acid or a salt thereof or a sulfite may be added to the aqueous phase to serve as an antioxidant in order to improve the antioxidation property of the water-soluble color in the liposomes.

The medium to serve as the aqueous phase to contain the substance to be encapsulated in accordance with the present invention may be distilled water, purified water, deep sea water or ultrafiltrates of deep sea water. In particular, the deep sea water, which is known to facilitate the growth of fibrocytes, can provide liposomes that not only have improved percutaneous absorption but are also capable of enhancing cell growth.

In the method of the present invention, liposomes are produced by adding the aqueous phase to the mixture of supercritical carbon dioxide and phospholipid. In doing so, the aqueous phase containing the substance to be encapsulated may be introduced into a homogenous fluid mixture consisting of carbon dioxide under the supercritical condition or under the condition in which temperature or pressure is higher or equal to its critical point, the phospholipid or the glycolipid, and optionally, the cosolvent, while the fluid mixture is vigorously stirred. As the aqueous phase is introduced, the initially clear homogenous fluid mixture gradually becomes turbid and turns whitish (due to formation of reversed micelles). As the aqueous phase is continuously introduced, the saturation point of water in the supercritical carbon dioxide (and reversed micelles) is eventually reached, upon which a turbid whitish aqueous layer is formed at the bottom of the reaction vessel. Once a predetermined amount of the aqueous phase has been injected, the pressure is reduced to form uniform liposome.

In accordance with the method of the present invention, unilamellar liposomes, 50nm to 800nm in diameter and with a high trapping efficiency of 7 to 30%, can be produced.

Consisting of fewer membranes, the liposomes produced in accordance with the method of the present invention resemble the biological membranes and can serve as a useful model for the biological membranes. Furthermore, the liposomes of the present invention are free of harmful organic solvents and thus do not exhibit the toxicity caused by the residual organic materials. Accordingly, the liposomes of the present invention are suitable for use as matrix to prevent disappearance of active ingredients of perfumes and cosmetics, preparations to facilitate percutaneous absorption of active ingredients, matrix to prevent inactivation of active ingredients of drugs, and DDS formulations to significantly reduce the risk of side effects.

The apparatus and the method for producing liposome in accordance with the present invention will now be described with reference to the accompanying drawings.

### BRIEF DESCRIPTION OF THE DRAWINGS

Fig. 1 is a schematic diagram showing one example of a first embodiment of an apparatus of the present invention.
Fig. 2 is a schematic diagram showing one example of a second embodiment of an apparatus of the present invention.
Fig. 3 is a schematic diagram showing of an apparatus which is not part of the present invention.
Fig. 4 is a schematic diagram showing a conventional method for producing liposome using supercritical carbon dioxide technique.
Fig. 5 is a photographic image by transmission electron micrography of liposomes prepared in Example 1.
Fig. 6 is a photographic image by transmission electron micrography of liposomes prepared in Example 2.
Fig. 7 is a photographic image by transmission electron micrography of liposomes prepared in Example 3.
Fig. 8 is a graphical representation showing the relationship between the lipid concentrations and the trapping efficiencies of liposomes prepared in Example 4.

### REFERENCE NUMERALS

- 1: Pressure reactor
- 2: Stirrer
- 3: Pump
- 4: Relief valve
- 5: Liposome
- 6: Receiver
- 7: Pump
- 8: Pump

### BEST MODES FOR CARRYING OUT THE INVENTION

### First embodiment

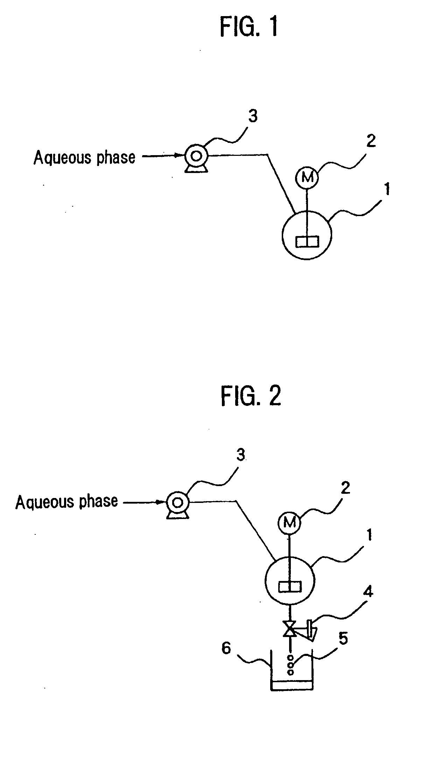

Referring to Fig. 1, a schematic diagram of one example of a first embodiment of an apparatus of the present invention is shown.

In this embodiment, a homogenous fluid mixture of supercritical carbon dioxide, a phospholipid or a glycolipid, and a cosolvent is first placed in a pressure reactor 1 under pressure. While the pressurized homogenous fluid mixture is being stirred vigorously with a stirrer 2, an aqueous phase containing a substance to be encapsulated is added dropwise into the pressure reactor 1 via a pump 3, which serves as means for introducing aqueous phase. Once a predetermined amount of the aqueous phase has been added, the pressure is released and the pressure reactor 1 opened to obtain liposomes encapsulating the substance.

### Second embodiment

Referring to Fig. 2, a schematic diagram of one example of a second embodiment of the present invention is shown. Elements analogous to those shown in Fig. 1 are designated by like reference numerals.

Again in this embodiment, a homogenous fluid mixture of supercritical carbon dioxide, a phospholipid or a glycolipid, and a cosolvent is first placed in a pressure reactor 1 under pressure. While the pressurized homogenous fluid mixture is being stirred vigorously with a stirrer 2, an aqueous phase containing a substance to be encapsulated is added dropwise into the pressure reactor 1 via a pump 3, which serves as means for introducing aqueous phase.

Unlike the first embodiment, a relief valve 4 is provided to serve as means for discharging liposomes. When the pressure in the pressure reactor 1 reaches a predetermined pressure or higher, liposome 5 is discharged from the pressure reactor 1 through the relief valve 4 into a receiver 6 and accumulates in the receiver 6.

### Third embodiment [NOT PART OF THE INVENTION]

Referring to Fig. 3, a schematic diagram of another example of the second embodiment of the present invention is shown. Elements analogous to those shown in Fig. 2 are designated by like reference numerals.

In Fig. 3, reference numeral 8 denotes a pump that serves as means for introducing a phospholipid or a glycolipid uniformly dispersed in a cosolvent into a pressure reactor 1, whereas reference numeral 9 denotes a pump that serves as means for introducing an aqueous phase containing a water-soluble or hydrophilic substance to be encapsulated.

In this embodiment, the supercritical carbon dioxide, the phospholipid or the glycolipid and the cosolvent, and the aqueous phase are introduced at once through the pumps 7, 8, and 3, respectively, into the pressure reactor 1. The mixture is then stirred with a stirrer 2 to form liposomes.

When the pressure in the pressure reactor 1 reaches a predetermined pressure or higher, liposome 5 is discharged from the pressure reactor 1 through a relief valve 4 into a receiver 6 and accumulates in the receiver 6.

The apparatus according to this embodiment permits continuous production of liposomes.

### Examples

### Example 1 and Comparative Example 1

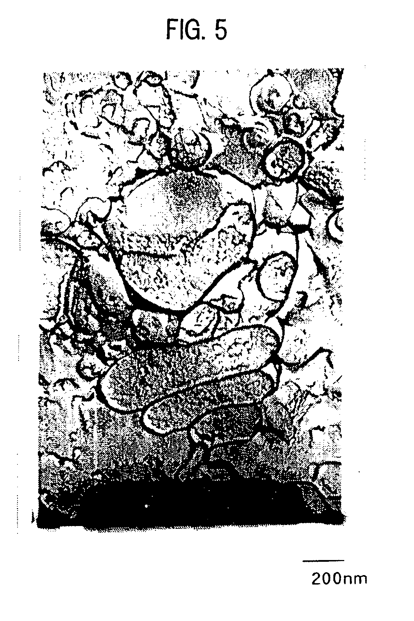

Liposomes were prepared using the apparatus shown in Fig. 1. The variable volume cell (inner volume of the cell = 57.02cm³) made by SUS304 and having a sapphire window was used as the pressure reactor. A fluid mixture of 0.014g dipalmitoylphosphatidylcholine (DPPC) and 13.749g supercritical carbon dioxide (200atm, 60°C) was placed in the pressure reactor. While the fluid mixture was being stirred vigorously, 2.81ml of a 3.5% aqueous solution of magnesium L-ascorbyl phosphate (VC-PMG) was injected into the pressure reactor with a pump at a flow rate of 0.05cc/min to prepare liposomes encapsulating VC-PMG. Morphology of the resulting liposomes was observed by transmission electron microscopy (TEM). The observation revealed that these liposomes were large unilamellar vesicles sized 100 to 1000nm. A TEM image of the liposomes was shown in Fig. 5.

The trapping efficiency of the liposomes was determined by the glucose dialysis, a standard technique. As Comparative Example 1, additional liposomes were prepared by sonication (Bangham method), which is also a commonly used technique in the preparation of liposomes, and the trapping efficiency of the liposomes was also determined. The results are shown in Table 1.

**Table 1**

| | Example 1 (supercritical liposomes) | Comparative Example 1 (liposomes made by Bangham method) |
|---|---|---|
| Trapping efficiency | 32% | 2.8% |

As can be seen from the results of Table 1, the liposome of Example 1 in the present invention, which makes use of supercritical carbon dioxide, has a trapping efficiency 10 or more times higher than that of the liposome of Comparative Example 1 prepared by the conventional sonication technique.

### Example 2

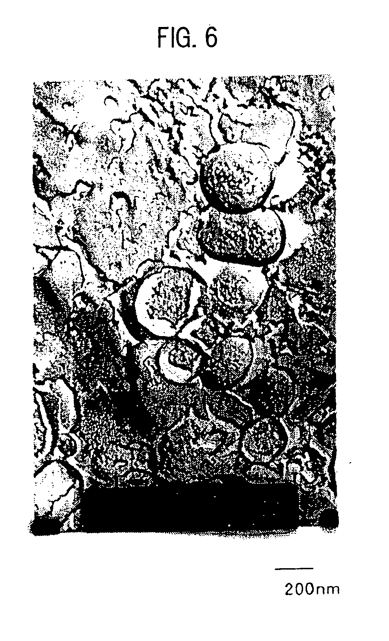

Liposomes were prepared using the apparatus shown in Fig. 2. The pressure reactor used was identical to that used in Example 1. A fluid mixture of 0.014g hydrogenated soy lecithin, 13.749g supercritical carbon dioxide (200atm, 60°C) and 0.96g ethanol was placed in the pressure reactor. While the fluid mixture was being stirred vigorously, 2.81ml of a 3.5% aqueous solution of magnesium L-ascorbyl phosphate (VC-PMG) was injected into the pressure reactor with a pump at a flow rate of 0.05cc/min. With the relief valve set to operate at 200kgf/cm², liposomes encapsulating VC-PMG were obtained. Morphology of the resulting liposomes was observed by transmission electron microscopy (TEM). The observation revealed that these liposomes were large unilamellar vesicles sized 100 to 1000nm. A TEM image of the liposomes was shown in Fig. 6.

### Example 3 and Comparative Example 2

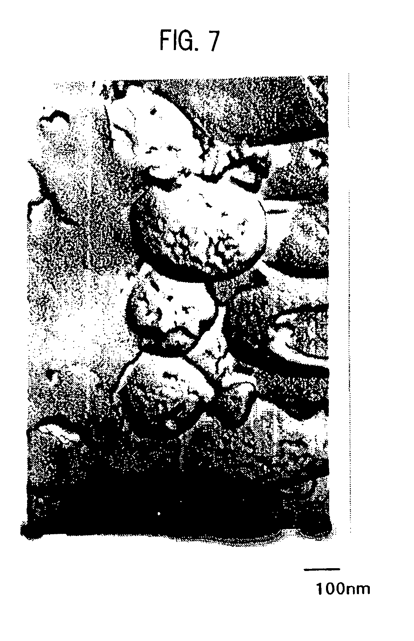

Liposomes were prepared using the apparatus shown in Fig. 2. The pressure reactor used was identical to that used in Example 1. A fluid mixture of 0.041g hydrogenated soy lecithin, supercritical carbon dioxide (200atm, 60°C) and 0.96g ethanol was placed in the pressure reactor. While the fluid mixture was being stirred vigorously, an aqueous solution of 10mg ibuprofen dissolved in 2.81ml water was injected into the pressure reactor with a pump at a flow rate of 0.05cc/min.
With the relief valve set to operate at 200kgf/cm², liposomes encapsulating ibuprofen were obtained. Morphology of the resulting liposomes was observed by transmission electron microscopy (TEM). The observation revealed that these liposomes were large unilamellar vesicles sized 100 to 1000nm. A TEM image of the liposomes was shown in Fig. 7.

The resulting liposomes were subjected to ultracentrifugation (45000rpm) to collect the aqueous phase entrapped within the liposomes, and the amount of the agent was determined. As Comparative Example 2, additional liposomes were prepared by sonication (Bangham method), which is also a commonly used technique in the preparation of liposomes, and the amount of the agent entrapped within the liposomes was also determined. The results are shown in Table 2.

**Table 2**

| | Example 3 (supercritical liposomes) | Comparative Example 2 (liposomes made by Bangham method) |
|---|---|---|
| Content of agent | 7.8mg | 0.6mg |

As can be seen from the results of Table 2, the liposome of Example 3 in the present invention, which makes use of supercritical carbon dioxide, contains 10 or more times as much agent as the liposome of Comparative Example 1 prepared by the conventional sonication technique.

### Example 4

Using the same apparatus as in Example 1, liposomes were prepared using the following conditions.

### Conditions for preparation

Temperature = 60°C; Pressure = 200bar; Lipid = 0.0353g; ethanol (for dissolving lipid) = 0.7963g; CO₂ = 12.1849g; flow rate of glucose solution = 0.1ml/min
Lipids used:
dioleoylphosphatidylcholine (DOPC)
dipalmitoylphosphatidylcholine (DPPC)
distearoylphosphatidylcholine (DSPC)

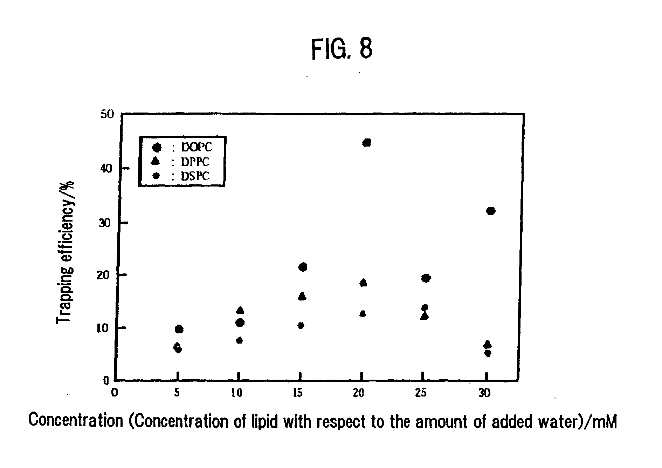

Three different types of supercritical liposomes were prepared using the three types of lipids and the conditions indicated above. The trapping efficiency was determined for each type of liposome. The results were plotted in a graph in Fig. 8.

The graph, with the vertical axis representing the trapping efficiency (%) and the horizontal axis representing the concentrations of the lipids with respect to the amount of water added, shows that each of the three types of liposome had significantly higher trapping efficiency as compared to the liposome prepared by the conventional Bangham method (trapping efficiency = 2 to 3%).

### INDUSTRIAL APPLICABILITY

The method and the apparatus of the present invention enable single-step, efficient production of liposomes with high trapping efficiency. The high trapping efficiency of the liposome produced by the method of the present invention facilitates percutaneous absorption of the encapsulated agent when the liposome is used in an external preparation and improves absorption of the encapsulated agent by body when the liposome is used for internal use.

By using deep sea water or its filtrates obtained by reverse osmosis membrane filtration as a media for aqueous phase, liposomes that facilitate the cell growth and percutaneous absorption of encapsulated agents can be obtained.

## Claims

1. A method for producing liposome encapsulating a desired substance, **characterized in that**:
an aqueous phase containing the substance to be encapsulated is added to a homogenous fluid mixture of a phospholipid or a glycolipid and carbon dioxide either under a supercritical condition or under a condition in which temperature or pressure is higher than or equal to its critical point
while adding the aqueous phase said condition is kept by maintaining the pressure and the homogenous fluid mixture is vigorously stirred;
and, once a predetermined amount of the aqueous phase has been added, the pressure is released to produce the liposomes.

2. The method for producing liposome according to claim 1, **characterized in that** the fluid mixture contains a cosolvent.

3. The method for producing liposome according to claim 1 or 2, **characterized in that** the aqueous phase contains deep sea water or its filtrate obtained by reverse osmosis membrane filtration.

4. The method for producing liposome according to any one of claims 1 to 3, **characterized in that** the substance to be encapsulated includes at least one water-soluble or hydrophilic pharmacologically active agent.

5. The method for producing liposome according to any one of claims 1 to 3, **characterized in that** the substance to be encapsulated includes at least one water-soluble color.

6. The method for producing liposome according to claim 5, **characterized in that** the aqueous phase contains as an antioxidant ascorbic acid or a salt thereof or a sulfite.

## Patentansprüche

1. Verfahren zur Herstellung von Liposomen, die eine gewünschte Substanz umschließen, **dadurch gekennzeichnet, daß**
eine wäßrige Phase, die die zu umschließende Substanz enthält, einem homogenen Flüssigkeitsgemisch als Phosphatids oder eines Glykollipids und Kohlenstoffdioxid entweder unter einer superkritischen Bedingung oder unter einer Bedingung zugesetzt werden, bei der die Temperatur oder der Druck höher als oder gleich ihrem kritischen Punkt sind, wobei während des Zusetzens der wäßrigen Phase diese Bedingung aufrechterhalten wird, indem der Druck und das homogene Flüssigkeitsgemisch heftig gerührt werden und daß, sobald eine vorbestimmte Menge der wäßrigen Phase zugesetzt worden ist, der Druck zur Erzeugung der Liposomen entspannt wird.

2. Verfahren zur Herstellung von Liposomen nach Anspruch 1, **dadurch gekennzeichnet, daß** das Flüssigkeitsgemisch ein Lösungsmittel enthält.

3. Verfahren zur Herstellung von Liposomen nach Anspruch 1 oder 2, **dadurch gekennzeichnet, daß** die wäßrige Phase Tiefseewasser oder sein Filtrat enthält, das durch Umkehrosmose-Membranhydration erhalten wird.

4. Verfahren zur Herstellung von Liposomen nach einem der Ansprüche 1 bis 3, **dadurch gekennzeichnet, daß** die Substanz, die zu umschließen ist, wenigstens ein wasserlösliches oder hydrophiles, pharmakologisch aktives Mittel enthält.

5. Verfahren zur Herstellung von Liposomen nach einem der Ansprüche 1 bis 3, **dadurch gekennzeichnet, daß** die zu umschließende Substanz wenigstens eine wasserlösliche Farbe aufweist.

6. Verfahren zur Herstellung von Liposomen nach Anspruch 5, **dadurch gekennzeichnet, daß** die wasserlösliche Phase eine antioxidante Ascorbinsäure oder ein Salz davon oder ein Sulfit enthält.

## Revendications

1. Procédé de production de liposomes encapsulant une substance désirée, **caractérisé par le fait que** :
une phase aqueuse contenant la substance à encapsuler est ajoutée à un mélange fluide homogène d'un phospholipide ou d'un glycolipide et de dioxyde de carbone soit dans un état supercritique soit dans un état dans lequel la température ou la pression est supérieure ou égale à son point critique ;
tout en ajoutant la phase aqueuse, ledit état est conservé par maintien de la pression et le mélange fluide homogène est agité énergiquement ;
et, une fois qu'une quantité prédéterminée de la phase aqueuse a été ajoutée, la pression est relâchée pour produire les liposomes.

2. Procédé de production de liposomes, selon la revendication 1, **caractérisé par le fait que** le mélange fluide contient un cosolvant.

3. Procédé de production de liposomes, selon l'une des revendications 1 ou 2, **caractérisé par le fait que** la phase aqueuse contient de l'eau de mer puisée en profondeur ou son filtrat obtenu par filtration sur membrane d'osmose inverse.

4. Procédé de production de liposomes, selon l'une quelconque des revendications 1 à 3, **caractérisé par le fait que** la substance à encapsuler comprend au moins un agent pharmacologiquement actif soluble dans l'eau ou hydrophile.

5. Procédé de production de liposomes, selon l'une quelconque des revendications 1 à 3, **caractérisé par le fait que** la substance à encapsuler comprend au moins un colorant soluble dans l'eau.

6. Procédé de production de liposomes, selon la revendication 5, **caractérisé par le fait que** la phase aqueuse contient, comme anti-oxydant, de l'acide ascorbique ou un sel de celui-ci ou un sulfite.
